# EUROPÄISCHE PATENTSCHRIFT

(11) **EP 1 846 055 B1**
(45) Veröffentlichungstag und Bekanntmachung des Hinweises auf die Patenterteilung: **31.12.2008**
(21) Anmeldenummer: 06700335.0
(22) Anmeldetag: 11.01.2006
(51) Int. Cl.: A61L 27/36, A61L 27/54

(54) **VERFAHREN ZUR HERSTELLUNG EINER OSTEOINDUKTIV-ANTISEPTISCHEN IMPLANTATBESCHICHTUNG**
METHOD FOR PRODUCING AN OSTEOINDUCTIVE-ANTISEPTIC IMPLANT COATING
PROCEDE DE PRODUCTION D'UN REVETEMENT D'IMPLANT OSTEOINDUCTIF-ANTISEPTIQUE

(30) Priorität: 14.01.2005 DE 102005002531
(43) Veröffentlichungstag der Anmeldung: 24.10.2007
(73) Patentinhaber: ESKA Implants GmbH & Co., 23556 Lübeck (DE)
(72) Erfinder: THOMAS, Wolfram, I-00135 Roma (IT); GRUNDEI, Hans, 23558 Lübeck (DE)
(74) Vertreter: Fuchs
(86) Internationale Anmeldenummer: PCT/EP2006/000162
(87) Internationale Veröffentlichungsnummer: WO 2006/077036

(56) Entgegenhaltungen:
- EP-A- 0 592 242
- US-A- 4 610 692
- US-A1- 2001 008 649
- US-B1- 6 485 754

## Beschreibung

Das Ziel jeder zementlosen Fixation von Endoprothesen ist eine möglichst schnelle und dauerhafte biologische Integration der Implantate: Dieser Einwachsvorgang wird durch die osteokonduktiven Eigenschaften der Verankerungsoberflächen der Endoprothesen ermöglicht. Bestimmte poröse Gestaltungen ermöglichen das An- bzw. Einwachsen von Knochen in die Öffnungen der Metallstrukturen.

Hydroxylapathit (HA) oder Tricalciumphosphat (TCP) als Beschichtungswerkstoffe dienen dem gleichen Ziel. Um diesen Vorgang zu garantieren und zu beschleunigen, können osteoinduktive Prozesse genutzt werden. Bekannte autologe Quellen osteoinduktiv wirksamer Materialien sind das Knochengewebe (besonders spongiöser Knochen) mit seinen Zellen und BMP's (Bone Morphogenic Proteins), das Knochenmark mit seinem Reichtum an Stammzellen und Osteoprogenitorzellen sowie thrombozytenreiches Plasma (PRP) mit seinen relevanten Wachstumsfaktoren. Mit dem Verfahren wird ein osteoinduktives Gel hergestellt, das aus den genannten drei autologen Quellen gespeist wird. Dieses Gel wird auf die Fixationsoberflächen von Endoprothesen aufgebracht und kann so nach Implantation in den Wirtsknochen seine osteoinduktive Potenz zur Beschleunigung des Einwachsprozesses entfalten (Biomaterialien 5 (4), "Osteoinduktives Gel zur Fixation von Endoprothesen", 2004, Seiten 249 ff.).

Darin wird vorgeschlagen, eine Methode zur osteoinduktiven Stimulation des Integrationsprozesses von Endoprothesen anzuwenden, die auch in anderen Fällen zur Beschleunigung der Osteoregeneration genutzt werden können wie beispielsweise bei Frakturen, Cysten, Defekten. Die wichtigsten osteoindukten Elemente sind Osteocyten, Osteoblasten und BMP's des Knochengewebes, Stammzellen und Osteoprogenitorzellen des Knochemarks und Wachstumsfaktoren (PDGF, IGF, TGF, EGF und VEGF) des thrombozytenreichen Plasmazentrifugates. Die Methode besteht in der zeitgleich zur Operation durchgeführten Herstellung des sogenannten osteoinduktiven Gels. Dieses osteoinduktive Gel wird aus drei Quellen gewonnen:

### 1. Autologer spongiöser Knochen:

Der Knochen wird bei der Herrichtung der Verankerungslager für die Endoprothesenimplantation-aus den Präzisionsfräsen entnommen und von evtl. vorhandenen Knorpelresten gereinigt. Dieser Knochenbrei beinhaltet die wichtigen osteoinduktiven Elemente Osteoblasten, Osteocyten und Bone Morphogenic Proteins (BMP's).

### 2. Platelet Rich Plasma (PRP):

Dieses Plasmazentrifugat enthält eine hohe Konzentration an Thrombozyten mit den relevanten Wachstumsfaktoren PDGF, IGF, TGF, EGF und VEGF.

Zur Herstellung des PRP eignet sich das Osteokinverfahren. Hierbei werden beispielsweise 60 ml Venenblut in einem doppelten Zentrifugierungsvorgang zu ca. 6 ml thrombozytenreichem Plasmagel verarbeitet.

### 3. Knochenmark:

Aus den bei der Operation eröffneten Markhölen von Femur und/oder Tibia (je nach Operationsmethode und gewähltem Implantat) wird mit einem Nelaton-Katheder und/oder mit einem kleinen chirurgischen Löffel reichlich Knochenmark mit seinen Stammzellen und Osteoprogenitorzellen entnommen. Das Material aus diesen drei Quellen wird in einer Metallschale gesammelt und unter Beigabe von Fibrin zu einer gelartigen Masse angerührt.

Dieses osteoinduktive Gel kann jetzt je nach Indikation und Anforderung benutzt werden. Zur osteoinduktiven Funktionalisierung der Endoprothese wird dieses Gel frisch auf deren strukturierte Verankerungsoberflächen aufgebracht. Besonders bevorzugt werden zementlos zu verankernde Endoprothesen, weil deren offenmaschige dreidimensionale metallische Raumnetzstruktur (Spongiosametall - DE-C-41 06 971) nicht nur ausgezeichnete osteokonduktive Eigenschaften besitzen, sondern sich wegen dieser Charakteristik auch besonders gut als Träger für das osteoinduktive Gel eignen.

Auf glatten oder mikrostrukturierten Oberflächen kann das Gel nicht haften. Das osteoinduktive Gel wird bei Bedarf zusätzlich auf die Knochenlager aufgebracht, was besonders bei Miniimplantaten (Oberflächenersatz, metaphysäre Endoprothesen) und bei Revisionseingriffen angebracht ist. Die derart vorbereiteten Implantate werden dann mit den entsprechenden Instrumenten eingebracht und fixiert.

In der weiter oben angegebenen Veröffentlichung wird berichtet, dass die Methode der osteoinduktiven Stimulation der Endoprothesenfixation 30 mal angewandt worden ist, 26 mal bei Hüftendoprothesen, viermal bei Knieendoprothesen.

Bei den Hüftendoprothesen handelt es sich in 14 Fällten um primäre diaphysäre Endoprothesen, in 6 Fällen um Oberflächenersatz mit einer Kappenendoprothese, in 4 Fällen um metaphysäre Endoprothesen und in 2 Fällen um diaphysäre Revisionsoperationen. In allen Fällen wurde als azetabuläre Komponente eine Press-fit-Pfanne benutzt.

Bei den Knieendoprothesen wurden dreimal die modulare so genannte Schlittenendoprothese implantiert, einmal eine teilverkoppelte Gleitachsenendoprothese.

Fünfmal ist das osteoinduktive Gel für andere Indikationen angewandt worden: Zweimal zur Fixation des freien Sehnentransplantates bei Kreuzbandplastik und dreimal bei plurifragmentären Tibiafrakturen mit Verplattungsteosynthese. Es konnten alle Patienten nach 6 Wochen, 3 Monaten, 6 Monaten und 12 Monaten nachuntersucht werden. Die mittlere Nachuntersuchungs-Zeit lag bei 8 Monaten (3 Mon. - 18 Mon.). Der Funktionsscore nach Harris zeigte einen sehr schnellen Anstieg von im Mittel praeoperativ 43 Punkten auf durchschnittliche 92 Punkte bereits nach 3 Monaten, was der Bewertungsgruppe "ausgezeichnet" entspricht. Solche Werte sind in Studien ohne Anwendung des osteoinduktiven Gels erst nach 9-12 Monaten erreicht worden. Alle Endoprothesen wurden bei den durchgeführten Kontrollen röntgenologisch untersucht. Hierbei zeigte sich eine schnelle Osteointegration in den Belastungszonen der Implantate.

Wenn die Anwendung des osteoinduktiven Gels auch Anlass zu berechtigten Hoffnungen hinsichtlich der Beschleunigung der Osteointegration sind, so könnten Entzündungen den an sich überaus positiven Wirkungen der osteoinduktiven Beschichtung des Implantates entgegenwirken.

Vor diesem Hintergrund ist es Aufgabe der vorliegenden Erfindung, ein Verfahren zur Herstellung einer osteoinduktiv-antiseptischen Implantatbeschichtung anzugeben, mit dem die Gefahr von Infektionen mit großer Sicherheit gebannt wird. Darüber hinaus soll eine Verwendung von Subtanzen zur Herstellung eines osteoinduktiv-antiseptischen Implantates vorgeschlagen werden mit ebenfalls geringstem Infektionsrisiko.

Desweiteren soll ein Set zur Erstellung eines künstlichen Implantates mit einer osteoinduktiv-antiseptischen Oberfläche vorgeschlagen werden, welches an die Kliniken zum intraoperativen Einsatz ausgeliefert werden kann.

Diese Aufgabe wird hinsichtlich des Verfahrens gelöst durch ein Verfahren mit den Merkmalen des Anspruchs 1. Hinsichtlich der Verwendung von Substanzen wird die Aufgabe gelöst durch die Verwendung gemäß Anspruch 7 zur Herstellung eines osteoinduktiv-antiseptisch beschichteten Implantates. Die Lösung hinsichtlich des Sets gelingt mit den Merkmalen des Anspruchs 12. Weitere vorteilhafte Varianten bzw. Ausführungsformen sind in den jeweiligen Unteransprüchen angegeben.

Verfahrensmäßig wird vorgeschlagen, dass zusätzlich zum PRP und autologem Knochenmark für die osteoinduktiv-antiseptische Implantatbeschichtung steriles Knochenmaterial von einer Knochenbank in Form eines feinkörnigen Granulats, ein Fibrinkleber und ein Antibiotikum zu den Substanzen zugegeben wird. Die Feinkörnigkeit des Granulats aus sterilem Knochenmaterial richtet sich nach der Größe der Hinterschneidungen auf der Oberfläche des Implantates. Sie muss so gewählt sein, dass die Granulatpartikel ohne weiteres hinter die Hinterschneidungen verbracht werden können durch Verstreichen der Substanz auf der Oberfläche des Implantates bzw. in diese hinein. Fibrinkleber verleiht der Masse eine streichfähige pastöse Substanz. Die pastöse Masse wird nach der Vermengung der Substanzen beispielsweise mit einem Spachtel in und auf die Oberfläche des Implantates aufgestrichen und das Implantat sodann am vorgesehenen Implantationsort implantiert. Überraschend hat sich gezeigt, dass eine Entzündungsgefahr des umgebenden Knochens (Gewebes) praktisch auszuschließen ist aufgrund der antiseptischen Wirkung des Antibiotikums und aufgrund der durch den Fibrinkleber verliehenen hohen Viskosität der Beschichtungsmasse, die ihr das notwendige örtliche Beharrungsvermögen verleiht. Das Antibiotikum sichert gegen eine Verkeimung üblicherweise nur für eine kurze Zeit. Dies reicht in der Praxis aber vollkommen aus. Mit dem erfindungsgemäß hergestellten beschichteten Implantat wird das Ziel erreicht, die Osteoinduktion mit der Osteokonduktion sowie mit der aseptischen Wirkung des Antibiotikums zu kombinieren.

Gemäß einer vorteilhaften Weiterbildung ist vorgesehen, dass die zugefügten Substanzen sich in ihren volumetrischen Mengenverhältnissen (V) V (PRP) : V (Fibrinkleber) : V (steriles Knochenmaterial) : V (Knochenmark) : V (Antibiotikum) verhalten wie 6 : 5 : 10 : 10 : 1. Diese Mengenverhältnisse rufen eine hervorragend geeignete Konsistenz der Beschichtungsmasse hervor bei gleich bleibend hoher antiseptischer Wirkung des Antibiotikums. Das verwendete Antibiotikum ist vorteilhafterweise hochkonzentriert.

In konkreter Anwendung ist vorgesehen, dass 6 ml PRP, 5 ml Fibrinkleber, 10 ml steriles Knochenmaterial, 10 ml Knochenmark und 2 ml Antibiotikum verarbeitet werden.

Bevorzugt ist die Verwendung einer Gentamycinlösung als Antibiotikum. Hochkonzentriert heißt für eine Gentamycinlösung in diesem Zusammenhang bevorzugt, dass in den 2 ml Gentamycinlösung 80 mg konzentriertes Gentamycin erhalten sind.

Alternativ kann eine Tobramycinlösung als Antibiotikum verwendet werden, oder aber eine Vancomycinlösung bzw. eine Kombination beider Antibiotika. Die erwähnten Antibiotika sind hochwirksam gegen die zur Zeit am meist verbreitesten Keime in der Orthopädie.

Hinsichtlich der Verwendung von Substanzen zur Herstellung eines osteoinduktiv-antiseptisch beschichteten Implantates ist vorgesehen, dass die Substanzen thrombozytenreiches Plasma (PRP), autologes Knochenmark, steriles Knochenmaterial von der Knochenbank in Form eines feinkörnigen Granulats, Fibrinkleber und ein Antibiotikum umfassen. Die Feinkörnigkeit des Granulats aus sterilem Knochenmaterial richtet sich nach der Größe der Hinterschneidungen auf der Oberfläche des Implantates. Sie muss so gewählt sein, dass die Granulatpartikel ohne weiteres hinter die Hinterschneidungen verbracht werden können durch Verstreichen der Substanz auf der Oberfläche des Implantates bzw. in diese hinein. Besonders bevorzugt wird, wenn sich die verwendeten Substanzen in ihren volumetrischen Mengenverhältnissen (V) V (PRP) : V (Fibrinkleber) : V (steriles Knochenmaterial) : V (Knochenmark) : V (Antibiotikum) verhalten wie 6 : 5 : 10 : 10 : 1.

Besonders bevorzugt kommt auch hier als Antibiotikum bevorzugt Gentamycinlösung, Tobramycinlösung oder Vancomycinlösung oder deren Kombination zum Einsatz.

Hinsichtlich des Sets zur Erstellung eines künstlichen Implantates mit einer wenigstens abschnittsweise osteoinduktiv-antiseptisch wirkenden Beschichtung wird vorgeschlagen, dass es umfasst: das Implantat mit einer wenigstens abschnittsweise Hinterschneidungen aufweisenden Oberfläche, vorzugsweise mit einer offenmaschigen dreidimensionalen Raumnetzstruktur, steriles Knochenmaterial von der Knochenbank in Form eines feinkörnigen Granulats, Fibrinkleber und ein Antibiotikum.

Die Feinkörnigkeit des Granulats aus sterilem Knochenmaterial richtet sich nach der Größe der Hinterschneidungen auf der Oberfläche des Implantates. Sie muss so gewählt sein, dass die Granulatpartikel ohne weiteres hinter die Hinterschneidungen verbracht werden können durch Verstreichen der Substanz auf der Oberfläche des Implantates bzw. in diese hinein.

Auch bei dem erfindungsgemäßen Set kommt als Antibiotikum vorzugsweise Gentamycinlösung, Tobramycinlösung oder Vancomycinlösung oder deren Kombination zum Einsatz.

Ein System zur Herstellung von thrombozytenreichem Plasma (PRP) muss bei dem Einsatz des Sets klinikseitig zur Verfügung stehen. Das autologe Knochenmark wird intraoperativ gewonnen.

Grundsätzlich gelten bei der Verwirklichung einer biologischen Fixation von Endoprothesen ohne Zement die Bedingungen der knöchernen Regeneration.

Das ideale Modell zur Anregung der Gestaltung von Implantaten und ihrer Fixation ist hierbei das Knochengewebe. Seine mechanische Resistenz garantiert die biomechanische Stabilität. Die Oberfläche und Porosität definieren seine osteokonduktiven Eigenschaften. Knochengewebe zeichnet sich aber besonders durch seine osteoindukte Aktivität der Bone Morphogenic Proteins (BMP's) und der Wachstumsfaktoren aus. Die regenerative Potenz wird noch verstärkt durch seine zellulären Elemente wie Osteoblasten und Osteocyten sowie Stammzellen und Osteoprogenitorzellen des Knochenmarks. Funktionelle Blutgefäße ermöglichen eine schnelle Vaskularisation des Regenerates und komplettieren die osteogenetische Qualität des Knochengewebes.

Ein Implantat zur zementlosen Fixation sollte unter Anlehnung an dieses biologische Modell eine Verankerungsoberfläche besitzen, die in ihrer Architektur, Struktur und Dimension möglichst genau die osteokonduktiven Eigenschaften des Knochens imitiert. Aus diesem Grunde werden bevorzugt Implantate mit Spongiosametalloberfläche benutzt, ein Fixationsprinzip, das seit nunmehr 20 Jahren klinisch sehr erfolgreich seine osteokonduktiven Qualitäten bestätigt hat. Spongiosametall besitzt eine Maschenweite von 500-1500 µm und ermöglicht damit das Durchwachsen stabiler, vaskularisierter Knochentrabekel zur definitiven Fixation.

Auf der Suche nach einer Beschleunigung dieses Einwachsprozesses durch osteinduktive Stimulation wurden zwei gut zugängliche Quellen autologen Materials identifiziert:

### 1. Platelet Rich Plasma (PRP):

Thrombocytenangereichertes Plasma ist eine reiche Quelle relevanter Wachstumsfaktoren
Platelet Derived Growth Factores - PDGF
Transforming Growth Factores - TGF
Epithelial Growth Factores - EGF
Insulin-like Growth Factores - IGF
Vascular Endothelial Growth Factores - VEGF

Die praktische Wirksamkeit dieses PRP wurde hauptsächüch in Tier versuchen dargestellt und hat inzwischen eine breite Anwendung in der Dental- und Maxillofazial-Chirurgie gefunden.

Bevorzugt wird das PRP nach Herstellung aus einem Zentrifugierungsverfahren mit dem Osteokin-System.

### 2. Knochenmark

Eine weitere Quelle osteoinduktiven Materials ist das Knochenmark, reich an Wachstumsfaktoren, Stammzellen und Osteoprogenitorzellen. Die osteoinduktive Potenz des Knochenmarks ist aus verschiedenen Tierstudien bekannt. Es existieren klinische Arbeiten über die Wirksamkeit von Knochenmarksfaktoren bei notwendigen Rekonstruktionen in der Revisionschirurgie von Endoprothesen. Es wurde im Rahmen des erfindungsgemäßen Verfahrens aus den osteoinduktiven Faktoren dieser drei Quellen das osteoinduktive Gel hergestellt und bei der zementlosen Fixation von Endoprothesen angewandt. Die dreidimensionale Struktur des Spongiosametalls zeigt sich hierbei durch seine makrostrukturelle Gestaltung besonders günstig als Träger für die Aufbringung des osteoinduktiven Gels.

Die Anwendung dieses osteoinduktiven Materials ist angezeigt bei der Primärimplantation von Endoprothesen, besonders unter dem Aspekt der immer häufiger angewendeten Minümplantate (minimalinvasive Endoprothetik), um deren primäre Osteointegration zu beschleunigen. Das osteoinduktive Gel hat sich aber auch besonders bewährt bei der Revisionsendoprothetik, wo oftmals knöcherne Defekte durch Osteolysen gefüllt werden müssen oder wo das leistungsschwache Knochenlager durch osteoinduktive Behandlung regeneriert werden kann.

Bei der klinischen Auswertung zeigt sich, dass die Patienten einen auffallend niedrigen Blutverlust haben, so dass die Rehabilitation ausgesprochen schnelle Fortschritte macht und Bluttransfusionen praktisch nicht nötig sind. Der deutlich schnellere Anstieg der Funktionsscores (innerhalb der ersten drei postoperativen Monate) im Vergleich zu Operationen ohne Wachstumsfaktoren ist ein Ausdruck der beschleunigten biologischen Fixation der Implantate. Auffallend ist, dass bislang keinerlei Komplikationen zu verzeichnen waren. Es wurden insbesondere keine heterotopen Ossifikationen beobachtet, weil die konzentriert vorliegenden Wachstumsfaktoren offensichtlich stabil im osteoinduktiven Gel eingebettet bleiben und nicht in die Umgebung abwandern können.

Es wird durch die Anwendung des osteoinduktiven Gels ein zusätzlicher in erster Linie auf die Beigabe von Antibiotikum beruhender antiinfektiver Effekt erwartet. Mit dieser Methode eröffnen sich auch andere Anwendungsmöglichkeiten, wie Auffüllung von Knochencysten oder ähnlicher Defekte, sowie die Behandlung von komplizierten Frakturen.

## Patentansprüche

1. Verfahren zur Herstellung einer osteoinduktiv-antiseptischen Implantatbeschichtung für ein Implantat mit einer wenigstens abschnittsweise Hinterschneidungen aufweisenden Oberfläche, bei dem thrombozytenreiches Plasma (PRP) sowie autologes Knochenmark miteinander vermischt werden, und bei dem der Masse zusätzlich steriles Knochenmaterial in Form eines feinkörnigen Granulats, ein Fibrinkleber und ein Antibiotikum zugegeben wird.

2. Verfahren nach Anspruch 1, **dadurch gekennzeichnet, dass** die zugefügten Substanzen sich in ihren volumetrischen Mengenverhältnissen (V) V (PRP) : V (Fibrinkleber) : V (steriles Knochenmaterial) : V (Knochenmark) : V (Antibiotikum) verhalten wie 6 : 5 : 10: 10: 2.

3. Verfahren nach Anspruch 2, **dadurch gekennzeichnet, dass** 6 ml PRP, 5 ml Fibrinkleber, 10 ml Knochen, 10 ml Knochenmark und 2 ml Antibiotikum verarbeitet werden.

4. Verfahren nach einem der Ansprüche 1 bis 3, bei dem als Antibiotikum Gentamycinlösung verwendet wird.

5. Verfahren nach einem der Ansprüche 1 bis 3, bei dem als Antibiotikum Tobramycinlösung verwendet.

6. Verfahren nach einem der Ansprüche 1 bis 3, bei dem als Antibiotikum Vancomycinlösung verwendet wird.

7. Verwendung von Substanzen zur Herstellung eines osteoinduktiv-antiseptisch beschichteten Implantates, mit einer wenigstens abschnittsweise Hinterschneidungen aufweisenden Oberfläche zur Behandlung eines knöchernen Defektes, bei dem die Substanzen neben thrombozytenreichem Plasma (PRP) und autologem Knochenmark umfassen:
- steriles Knochenmaterial in Form eines feinkörnigen Granulats,
- ein Fibrinkleber, und
- ein Antibiotikum

8. Verwendung nach Anspruch 7, **dadurch gekennzeichnet, dass** sich die verwendeten Substanzen in ihren volumetrischen Mengenverhältnissen (V) V (PRP) : V (Fibrinkleber) : V (steriles Knochenmaterial) : V (Knochenmark) : V (Antibiotikum) verhalten wie 6 : 5 : 10 : 10 :1.

9. Verwendung nach Anspruch 7 oder 8, bei dem als Antibiotikum Gentamycinlösung verwendet wird.

10. Verwendung nach Anspruch 7 oder 8, bei dem als Antibiotikum Tobramycinlösung verwendet wird.

11. Verwendung nach Anspruch 7 oder 8, bei dem als Antibiotikum Vancomycinlösung verwendet wird.

12. Set zur Erstellung eines künstlichen Implantates mit einer osteoinduktiv-antiseptisch wirkenden Beschichtung, umfassend:
- das Implantat mit einer wenigstens abschnittsweise Hinterschneidungen aufweisenden Oberfläche
- steriles Knochenmaterial in Form eines feinkörnigen Granulats,
- ein Fibrinkleber, und
- ein Antibiotikum.

13. Set nach Anspruch 12, bei dem als Antibiotikum Gentamycinlösung verwendet wird.

14. Set nach Anspruch 12, bei dem als Antibiotikum Tobramycinlösung verwendet wird.

15. Set nach Anspruch 12, bei dem als Antibiotikum Vancomycinlösung verwendet wird.

## Claims

1. A method for producing an osteoinductive-antiseptic implant coating for an implant having a surface exhibiting at least portionwise undercuts, wherein platelet-rich plasma (PRP) and autologous bone marrow are mixed together, and wherein sterile bone material in the form of a fine-grained granulate, and a fibrin glue and an antibiotic are additionally added to the mass.

2. A method according to Claim 1, **characterised in that** the added substances are in a volumetric ratio (V) of V (PRP) : V (fibrin glue) : V (sterile bone material) : V (bone marrow) : V (antibiotic) of 6 : 5 : 10 : 10: 2.

3. A method according to Claim 2, **characterised in that** 6 ml of PRP, 5 ml of fibrin glue, 10 ml of bone,10 ml of bone marrow and 2 ml of antibiotic are worked up.

4. A method according to any one of Claims 1 to 3, wherein gentamicin solution is used as the antibiotic.

5. A method according to any one of Claims 1 to 3, wherein tobramycin solution is used as the antibiotic.

6. A method according to any one of Claims 1 to 3, wherein vancomycin solution is used as the antibiotic.

7. A use of substances to produce an osteoinductive-antiseptically coated implant having a surface exhibiting at least portionwise undercuts for the treatment of a bony defect, wherein the substances include, in addition to platelet-rich plasma (PRP) and autologous bone marrow:
- sterile bone material in the form of a fine-grained granulate,
- a fibrin glue, and
- an antibiotic.

8. A use according to Claim 7, **characterised in that** the substances used are in a volumetric ratio (V) of V (PRP) : V (fibrin glue) : V (sterile bone material) : V (bone marrow) : V (antibiotic) of 6 : 5 : 10 : 10 : 1.

9. A use according to Claim 7 or 8, wherein gentamicin solution is used as the antibiotic.

10. A use according to Claim 7 or 8, wherein tobramycin solution is used as the antibiotic.

11. A use according to Claim 7 or 8, wherein vancomycin solution is used as the antibiotic.

12. A kit for producing an artificial implant having an osteoinductive-antiseptically acting coating, comprising:
- the implant having a surface exhibiting at least portionwise undercuts;
- sterile bone material in the form of a fine-grained granulate;
- a fibrin glue, and
- an antibiotic.

13. A kit according to Claim 12, wherein gentamicin solution is used as the antibiotic.

14. A kit according to Claim 12, wherein tobramycin solution is used as the antibiotic.

15. A kit according to Claim 12, wherein vancomycin solution is used as the antibiotic.

## Revendications

1. Procédé de production d'un revêtement ostéo-inducteur antiseptique pour un implant dont la surface présente au moins par endroits des contre-dépouilles, dans lequel du plasma riche en thrombocytes (PRP) ainsi que de la moelle osseuse autologue sont mélangés ensemble et dans lequel du matériau osseux stérile sous la forme d'un granulé à grain fin, une colle de fibrine et un antibiotique sont ajoutés en plus à la pâte.

2. Procédé selon la revendication 1, **caractérisé en ce que** les substances ajoutées sont présentes selon un ratio quantitatif en volume (V) V (PRP) : V (colle de fibrine) : V (matériau osseux stérile) : V (moelle osseuse) : V (antibiotique) égal par exemple à 6 : 5 : 10 : 10 : 2.

3. Procédé selon la revendication 2, **caractérisé en ce que** 6 ml de PRP, 5 ml de colle de fibrine, 10 ml d'os, 10 ml de moelle osseuse et 2 ml d'antibiotique sont mis en oeuvre.

4. Procédé selon l'une des revendications 1 à 3, dans lequel l'antibiotique utilisé est une solution de gentamycine.

5. Procédé selon l'une des revendications 1 à 3, dans lequel l'antibiotique utilisé est une solution de tobramycine.

6. Procédé selon l'une des revendications 1 à 3, dans lequel l'antibiotique utilisé est une solution de vancomycine.

7. Utilisation de substances pour produire un implant doté d'un revêtement ostéo-inducteur antiseptique et dont la surface présente au moins par endroits des contre-dépouilles, destiné au traitement d'un défaut osseux, dans laquelle les substances comprennent, outre du plasma riche en thrombocytes (PRP) et de la moelle osseuse autologue :
- du matériau osseux stérile sous la forme d'un granulé à grain fin,
- une colle de fibrine, et
- un antibiotique

8. Utilisation selon la revendication 7, **caractérisée en ce que** les substances ajoutées sont présentes selon un rapport quantitatif en volume (V) V (PRP) : V (colle de fibrine) : V (matériau osseux stérile) : V (moelle osseuse) : V (antibiotique) égal par exemple à 6 : 5 : 10 : 10 : 1.

9. Utilisation selon la revendication 7 ou la revendication 8, dans laquelle l'antibiotique utilisé est une solution de gentamycine.

10. Utilisation selon la revendication 7 ou la revendication 8, dans laquelle l'antibiotique utilisé est une solution de tobramycine.

11. Utilisation selon la revendication 7 ou la revendication 8, dans laquelle l'antibiotique utilisé est une solution de vancomycine.

12. Kit de réalisation d'un implant artificiel doté d'un revêtement ayant un effet ostéo-inducteur antiseptique, comprenant :
- l'implant avec une surface présentant au moins par endroits des contre-dépouilles,
- du matériau osseux stérile sous la forme d'un granulé à grain fin,
- une colle de fibrine, et
- un antibiotique.

13. Kit selon la revendication 12, dans lequel l'antibiotique utilisé est une solution de gentamycine.

14. Kit selon la revendication 12, dans lequel l'antibiotique utilisé est une solution de tobramycine.

15. Kit selon la revendication 12, dans lequel l'antibiotique utilisé est une solution de vancomycine.
